# EUROPEAN PATENT APPLICATION

(11) **EP 4 609 798 A1**
(43) Date of publication of application: **03.09.2025**
(21) Application number: 24160147.5
(22) Date of filing: 28.02.2024
(51) Int. Cl.: A61B 8/00, A61B 5/00

(54) **DEVICE FOR MOUNTING A SENSOR ON SKIN**

(71) Applicant: Koninklijke Philips N.V., 5656 AG Eindhoven (NL)
(72) Inventor: VAN ROOIJ, Johannes Antonius, Eindhoven (NL); VAN KESTEREN, Hans Willem, Eindhoven (NL)
(74) Representative: Philips Intellectual Property & Standards

(57) **Abstract**

A device for mounting a sensor on a subject's skin. The device comprises a resilient member, at least one adhesive layer for securing the device to the subject's skin, and a sensor-securing arrangement for securing a mounted sensor to the subject's skin. The resilient member deforms to provide a preload force when the device is secured to a subject. A skin-coupling element of the resilient member comprises a skin-coupling surface that is configured to bend in at least a first direction to conform to a curvature of the skin.

## Description

### FIELD OF THE INVENTION

The invention relates to the field of mounting sensors on the body.

### BACKGROUND OF THE INVENTION

Ultrasound transducers can be used in a variety of monitoring applications as sensors. For example, an ultrasound transducer may be used to: image an artery to measure the inner diameter of that artery using e.g. B-mode; and/or measure blood flow parameters of blood passing through that artery, using e.g. Color-Doppler Pulsed-Wave-Doppler. This can be done over an extended period of time (e.g. up to 72 hours) for the purpose of e.g. hemodynamic monitoring.

This requires an ultrasound transducer to be in acoustic contact with a desired area of a subject's skin for a period of several hours. This acoustic contact must be maintained over the required monitoring time, without causing undue discomfort to the subject.

Various techniques have been proposed for securing ultrasound transducers and other sensors to a subject's skin. These techniques generally involve the use of an adhesive. However, the use of an adhesive alone typically does not provide an appropriate coupling strength: if the adhesive is too weak, the sensor will become at least partially detached from the skin over time, and may detach completely; on the other hand, if the adhesive is too strong, the attachment of the sensor may cause discomfort, and even pain, to the subject during use of the sensor (e.g. when applied unevenly, the attachment may irritate the skin by pinching or stretching) and during removal of the sensor from the skin.

WO 2020/229589 A1 discloses an assembly for mounting a sensor on skin, comprising at least one resilient member configured to bias a surface of a sensor unit against the skin of a subject. This provides improved coupling between the sensor unit and the skin, because the resilient member provides a preload force that is sufficient to maintain good contact between the sensor and the skin over an extended period. This pre-load will be maintained as long as the adhesive(s) adheres to the skin.

However, an at least partial detachment of a sensor can still occur when using this assembly on certain areas of skin, in particular on areas having a high degree of curvature, such as the neck, arm and leg. These are locations that are commonly imaged for hemodynamic monitoring, in which it is important that an ultrasound transducer is able to maintain acoustic contact with the skin for several hours.

There is therefore a need for an improved device for mounting a sensor on a subject's skin.

### SUMMARY OF THE INVENTION

The invention is defined by the claims.

According to examples in accordance with an aspect of the invention, there is provided a device for mounting a sensor on a subject's skin, the device comprising:
a resilient member configured to deform to create a preload force between the device and the subject's skin, wherein the resilient member comprises a skin-coupling element having a skin-coupling surface configured to bend in at least a first direction; at least one adhesive layer for securing the device to the subject's skin; and a sensor-securing arrangement configured to secure a sensor to the subject's skin when the device is secured to the subject's skin.

A skin-coupling element of a resilient member is a part of the resilient member having a surface that engages with the skin (either by direct contact with the skin, or indirectly via an adhesive layer) when the device is secured to the skin.

The inventors have recognized that the use of a resilient member having a flat, rigid skin-coupling element on curved skin results in air gaps between the skin-coupling element and the skin. This causes a weaker attachment between the device and the skin, by reducing the peeling strength, and over time, can result in the device (and therefore the sensor mounted using the device) to become at least partially detached from the skin (e.g. by peeling away from the skin).

By configuring the skin-coupling surface of the resilient member to bend to conform to the curvature of a subject's skin, a better adherence between the device and the skin may be achieved. The peeling strength of the at least one adhesive layer is increased by reducing an angle between the adhesive and the skin, thus reducing a likelihood that the device will become detached from the skin during use, as the tensile or shear strength of the adhesive must instead be overcome. "Bend" is used in the present disclosure to refer to any change in curvature of the skin-coupling surface. In other words, the skin-coupling surface is able to deviate from a fixed curvature.

In addition to improving adherence, a skin-coupling element that conforms to the curvature of a subject's skin is less likely to stretch or pinch the skin, making the device more comfortable to wear.

The curvature required for optimal adherence to the subject's skin varies between subjects and between body parts of a subject. By using a skin-coupling surface that is able to bend (i.e. rather than a skin-coupling surface of a fixed curvature), the skin-coupling surface is able to better conform to a variety of skin curvatures.

In some examples, the skin-coupling element comprises at least one first region of a first rigidity and two or more second regions of a second rigidity higher than the first rigidity, wherein each first region is positioned between at least two of the second regions.

The higher-rigidity regions enable the device to support the weight of a sensor while the device is attached to the skin, while the at least one region of lower rigidity allows the skin-coupling surface to bend to conform to skin curvature.

In some examples, each first region is formed by a region of the skin-coupling element having a first thickness in a direction perpendicular to the skin-coupling surface of the skin-coupling element; and each second region is formed by a region of the skin-coupling element having a second thickness in the direction perpendicular to the skin-coupling surface of the skin-coupling element, wherein the second thickness is greater than the first thickness.

In other words, the skin-coupling element comprises thinner sections that act as hinges, allowing the skin-coupling surface to bend.

In some examples, the skin-coupling element is configured such that, when the device is secured to the subject's skin, an angle between the skin-coupling surface and the surface of the subject's skin to which the device is secured is zero.

When the angle between the skin-coupling surface and the skin surface is zero, the device cannot peel away from the skin. Instead, the much stronger shear or tensile forces would need to be overcome in order for the device to become detached from the skin. In some examples, the skin-coupling surface is further configured to bend in a second direction, perpendicular to the first direction.

In other words, the skin-coupling surface may be configured to bend biaxially. This allows the skin-coupling surface to curve in two directions, thus further improving a coupling of the skin-coupling surface with a curved area of skin.

In some examples, the resilient member comprises a plurality of interconnected ribs extending around an aperture, wherein the resilient member is configured to deform by moving between an undeformed state, in which the interconnected ribs are closer together, and a deformed state, in which the interconnected ribs are further apart.

In some examples, the resilient member comprises a pleated structure comprising a plurality of pleats, wherein the pleated structure is configured to deform by moving between an undeformed state, in which the plurality of pleats are contracted, and a deformed state, in which the plurality of pleats are expanded.

In some examples, the at least one adhesive layer comprises a first adhesive layer on the skin-coupling surface of the skin-coupling element.

In other words, the device may be secured to the subject's skin by an adhesive layer between the skin-coupling element and the skin.

In some examples, the at least one adhesive layer comprises a second adhesive layer provided around an outer periphery of the skin-coupling surface of the skin-coupling element.

This provides a greater area of adhesion between the device and the subject's skin.

In some examples, the device further comprises: an acoustic gel pad comprising a skin-coupling pad surface; and a housing for holding the acoustic gel pad.

An acoustic gel pad provides a reliable acoustic contact between a sensor mounted on the device and the subject's skin. In particular, the use of an acoustic gel pad is preferred over the use of liquid acoustic gel, as liquid gel would be smeared over the subject's skin while positioning the device, and further, liquid gel dries over time, making it less suitable for longitudinal monitoring.

In some examples, the skin-coupling pad surface is curved. In this way, an acoustic contact between the sensor and the skin is improved.

In some examples, the housing comprises a skin-coupling housing surface, wherein the skin-coupling housing surface is curved. This improves a comfort of a subject wearing the device.

In some examples, the at least one adhesive layer is attached to the resilient member; in an undeformed state of the resilient member, the skin-coupling surface of the resilient member is offset from the skin-coupling pad surface in a direction perpendicular to the skin-coupling pad surface by a first distance; and in a deformed state of the resilient member, an offset of the skin-coupling surface of the resilient member and the skin-coupling pad surface in a direction perpendicular to the skin-coupling pad surface is less than the first distance.

This allows the device to be moved across the surface of the subject's skin without adhering to the skin, in order to correctly position the device on the body. In particular, in the case of an ultrasound transducer, it enables an ultrasound transducer mounted on the device to be moved across the skin while in acoustic contact with the skin, allowing ultrasound imaging data from the ultrasound transducer to be used to position the device. By contrast, when using a device for which there is no distance between the adhesive and the skin when the device is placed on the skin, the device must be removed from the skin after the desired position is achieved, in order to remove the backing layer from the adhesive. This risks incorrect positioning of the device when the device is placed back on the skin.

Once a desired position is achieved, the resilient member may be deformed, creating a preload force between the device and the skin and bringing the at least one adhesive layer into contact with the skin.

In some examples, in a deformed state of the resilient member, the offset of the skin-coupling surface of the resilient member and the skin-coupling pad surface in a direction perpendicular to the skin-coupling pad surface is zero.

In other words, when the device is mounted on the skin and the resilient member is deformed, the skin-coupling pad surface and the skin-coupling surface of the resilient member are both in contact with the skin.

In some examples, the acoustic gel pad and the housing are configured to interlock to secure the acoustic gel pad to the housing.

By securing the acoustic gel pad to the housing in this way, the acoustic gel pad is more likely to remain within the housing when mounting the sensor onto the device and when moving the device across the skin.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an exploded view of a device for mounting a sensor on a subject's skin, according to an embodiment of the invention;
Fig. 2 illustrates a perspective top view of the assembled device;
Fig. 3 illustrates a perspective top view of an ultrasound transducer mounted in the device;
Fig. 4 illustrates a perspective bottom view of the device with the ultrasound transducer mounted in the device;
Fig. 5 illustrates a perspective top view of the resilient member in an undeformed state;
Fig. 6 illustrates a side view of the resilient member in a deformed state;
Fig. 7 illustrates a perspective side view of a device for mounting a sensor on a subject's skin, according to an embodiment of the invention;
Fig. 8 illustrates a sectional view of the device of Fig. 7;
Fig. 9 illustrates a top view of the acoustic gel pad and the housing; and
Fig. 10 illustrates a sectional view of the acoustic gel pad secured within the housing.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for mounting a sensor on a subject's skin. The device comprises a resilient member, at least one adhesive layer for securing the device to the subject's skin, and a sensor-securing arrangement for securing a mounted sensor to the subject's skin. The resilient member deforms to provide a preload force when the device is secured to a subject. A skin-coupling element of the resilient member comprises a skin-coupling surface that is configured to bend in at least a first direction, for example by using an elastic deformable element, to conform to a curvature of the skin.

Embodiments are at least partly based on the realization that a device having a resilient member with a flat, rigid skin-coupling surface does not remain adhered to curved skin for the duration of some imaging procedures, in particular, for longitudinal monitoring procedures, and that such a device may cause discomfort when worn on curved skin, due to stretching or pinching of the skin.

Illustrative embodiments may, for example, be employed in hemodynamic monitoring.

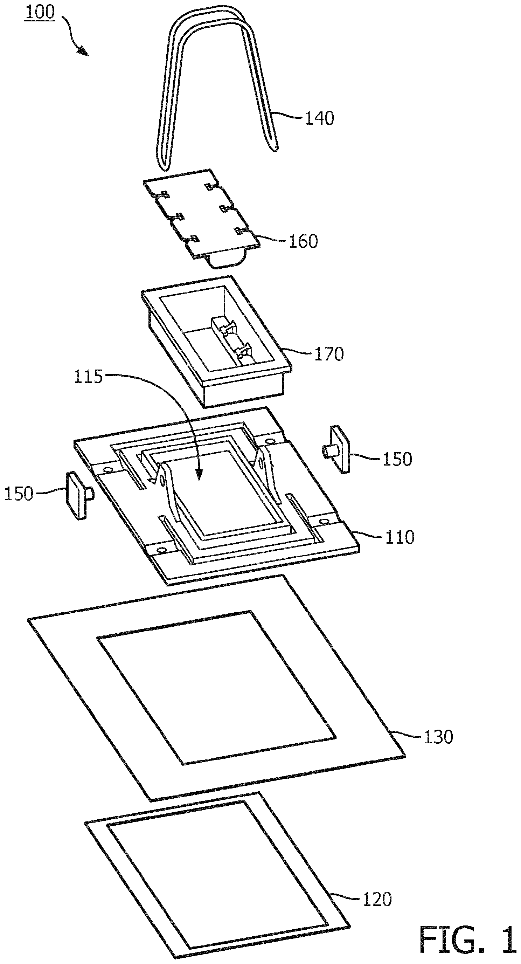

Fig. 1 illustrates an exploded view of a device 100 for mounting a sensor on a subject's skin, according to an embodiment of the invention. The device 100 comprises a resilient member 110, a first adhesive layer 120, a second adhesive layer 130, and a sensor-securing arrangement.

The resilient member 110 is configured to deform to create a preload force between the device 100 and a subject's skin, when the device is mounted on a subject. This ensures that the preload force between the device and the subject's skin is sufficiently strong to keep a sensor mounted on the device pressed against the skin for a duration of a procedure in which the sensor is used (e.g. a hemodynamic monitoring procedure).

In Fig. 1, the resilient member 110 comprises a plurality of interconnected ribs extending around an aperture 115 for containing a sensor. The resilient member is configured to deform by moving between an undeformed state, in which the interconnected ribs are closer together, and a deformed state, in which the interconnected ribs are further apart.

As the skilled person will appreciate, the invention is not limited to the particular resilient member illustrated in Fig. 1, and other types of resilient member suitable for use in the device 100 will be readily apparent to the skilled person. For instance, the resilient member may comprise a pleated structure comprising a plurality of pleats and configured to deform by moving between an undeformed state, in which the plurality of pleats are contracted, and a deformed state, in which the plurality of pleats are expanded. International Patent Application No. WO 2020/229589 A1 describes further resilient members that may be used in the device 100.

The first and second adhesive layers 120, 130 are configured to secure the device to a subject's skin. The first adhesive layer 120 is a double-sided adhesive layer (i.e. with adhesive on opposing surfaces of the layer), which, when the device 100 is assembled, is provided on a skin-coupling surface of the resilient member 110 (i.e. between the resilient member and the skin when the device is secured to a subject's skin). The second adhesive layer 130 is provided around an outer periphery of the skin-coupling surface of the resilient member.

Although the device 100 illustrated in Fig. 1 comprises both the first adhesive layer 120 and the second adhesive layer 130, in some examples, only one or other of the first and second adhesive layers may be used to secure the device to a subject's skin.

The sensor-securing arrangement in Fig. 1 comprises an elastic band 140 and two pins 150, provided on either side of the device 100 to secure the elastic band to the device. The resilient member 110 comprises two through-holes on opposing sides of the resilient member for attaching the pins to the resilient member.

Alternative sensor-securing arrangements that may be used in place of the elastic band 140 and pins 150 shown in Fig. 1 will be readily apparent to the skilled person. For instance, a sensor may be secured to the device using one or more clips or snap-fit connectors. In some examples, the resilient member may itself secure a sensor to a subject's skin when the device is secured to the subject's skin (e.g. the aperture 115 may be configured to provide a friction fit, or the resilient member may comprise an element configured to push a sensor towards the subject's skin).

In Fig 1, the device 100 further comprises an acoustic gel pad 160 and a housing 170 for holding the acoustic gel pad. The housing is configured to fit in the aperture 115 of the resilient member, and defines a space large enough to contain the acoustic gel pad 160. In some examples, the housing may not fully contain the acoustic gel pad; for instance, the acoustic gel pad may extend from an opening in the housing (e.g. an opening closest to the skin when the device is mounted on a subject). The housing may be made from plastic, and may be manufactured using any known plastic-manufacturing technique (e.g. injection molding, extrusion, etc.).

The acoustic gel pad 160 is formed from an acoustically translucent aqueous gel, and is configured to provide acoustic contact between a subject's skin and an ultrasound transducer mounted on the device. For instance, the acoustic gel pad may be formed from a combination of a gel, water and a lubricous material (e.g. 10% polyvinylpyrrolidone, 89% water 1% polyvinyl alcohol). Suitable materials for acoustic gel pads will be readily apparent to the skilled person. In some examples, the acoustic gel pad may be an Aquaflex^{®} ultrasound gel pad. The acoustic gel pad and the housing are described in more detail below.

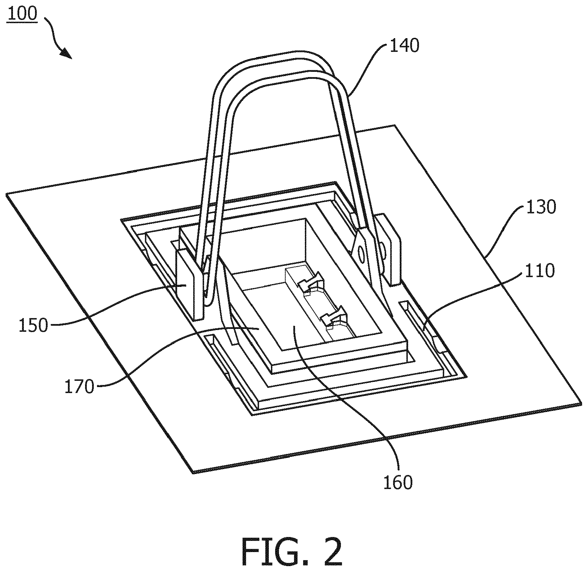

Fig. 2 illustrates a perspective top view of the assembled device 100. The housing 170 for the acoustic gel pad is inserted into the aperture 115 of the resilient member 110, and the acoustic gel pad 160 is contained within the housing. In Fig. 2, the acoustic gel pad does not fill the space defined by the housing; in this way, a sensor mounted on the device may be partially contained within the housing, allowing the sensor to be more securely attached to the device.

A part of the adhesive surface of the second adhesive layer 130 is affixed to a top surface (i.e. opposite the skin-coupling surface) of the resilient member. The second adhesive layer extends outwards from the resilient member, so that the remainder of the adhesive surface can be secured to a subject's skin. The first adhesive layer 120, affixed to the skin-coupling surface of the resilient member, is not visible in Fig. 2.

The pins 150 are inserted into the through-holes of the resilient member 110. In Fig. 2, each pin engages with a respective through-hole to provide a friction fit; however, the pins may be secured to the resilient member by other means (e.g. a stopper may be secured to an end of the pin once the end has passed through the through-hole). The elastic band 140 is looped around each pin to provide the sensor-securing arrangement.

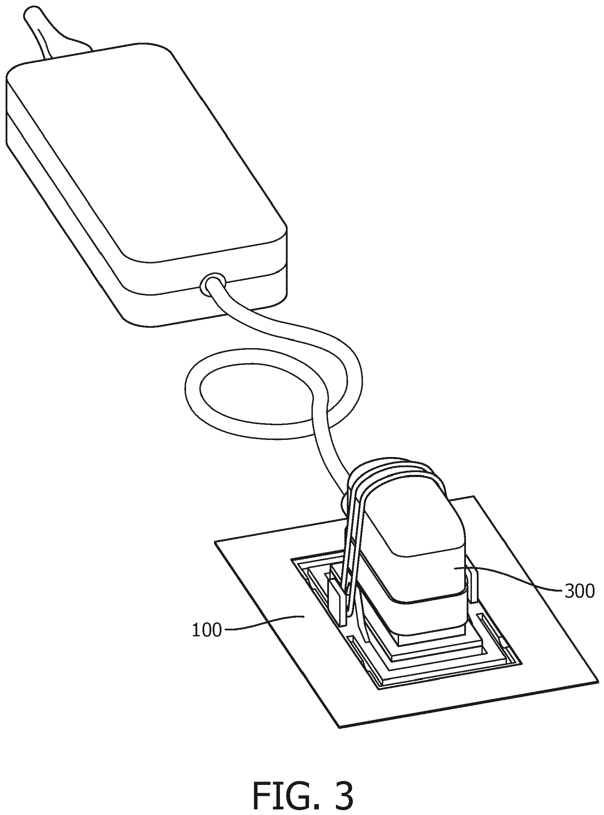

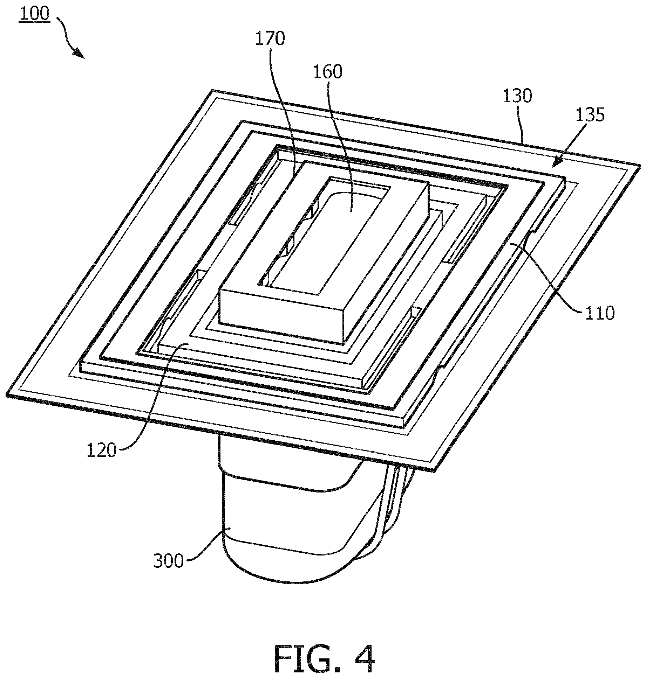

Fig. 3 illustrates a perspective view of an ultrasound transducer 300 mounted in the bottom view of the device 100 with the ultrasound transducer 300 mounted in the device. In Fig. 4, the device 100. The ultrasound transducer is partially contained within the housing 170, and is secured to the device by the elastic band 140. The elastic band provides a force that pushes the mounted ultrasound transducer towards the acoustic gel pad 160, improving the acoustic contact between the ultrasound transducer and the acoustic gel pad. An ultrasound-emitting surface of the ultrasound transducer faces towards the acoustic gel pad 160.

The ultrasound transducer shown in Fig. 3 is a type of ultrasound transducer suitable for hemodynamic monitoring . However, the device may be used with any type of ultrasound transducer (including linear array transducers, curved array transducers and sector array transducers).

Fig. 4 illustrates a perspective adhesive surface 135 of the second adhesive layer and the skin-coupling surface of the resilient member 110 are visible.

The skin-coupling surface of the resilient member 110 is a surface that engages with the skin (via the first adhesive layer) when the device is secured to a subject. The first adhesive layer 120 is provided on the skin-coupling surface of the resilient member. As previously mentioned, the first adhesive layer is a double-sided adhesive. An upper adhesive surface of the first adhesive layer is affixed to the skin-coupling surface of the resilient member. A lower adhesive surface of the first adhesive layer, on an opposing side of the first adhesive layer to the upper adhesive surface, adheres to the skin when the device is mounted on a subject.

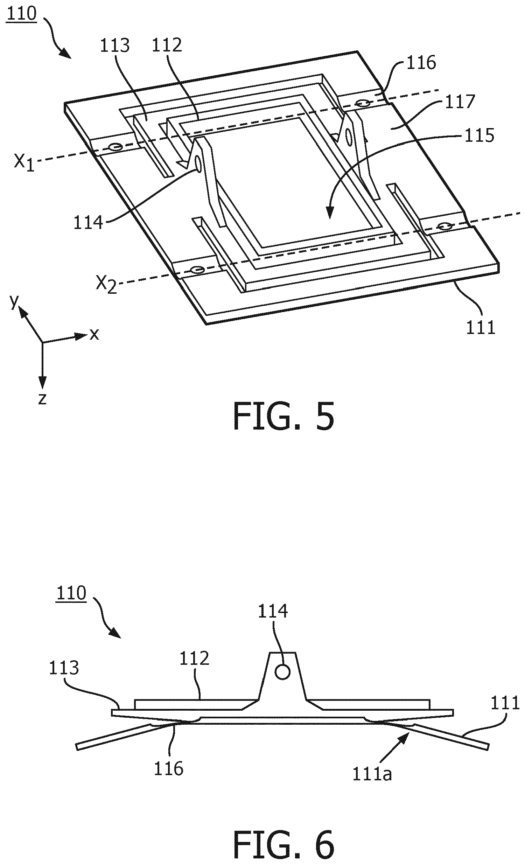

Fig. 5 illustrates a perspective top view of the resilient member 110 in an undeformed state. The resilient member comprises a skin-coupling element 111, an inner rim 112 and a deformable portion 113 provided between the skin-coupling element and the inner rim. The resilient member further comprises two elongated elements, extending from the resilient member, each comprising a through-hole 114 for inserting the pins 150 of the sensor-securing arrangement, as described above.

The deformable portion 113 comprises a plurality of interconnected ribs, as previously described. The inner rim 112 defines the aperture 115 into which the housing 170 for the acoustic gel pad is inserted.

In the undeformed state shown in Fig. 5, the skin-coupling element 111, inner rim 112 and deformable portion 113 lie in a same xy-plane. In a deformed state, the interconnected ribs of the deformable portion are separated in a z-direction, causing a separation, in the z-direction, of the skin-coupling element and the inner rim.

The skin-coupling surface of the resilient member (not visible in Fig. 5) is a surface of the skin-coupling element 111 that engages with the skin (via the first adhesive layer) when the device is mounted on a subject with the resilient member 110 in a deformed state.

The skin-coupling surface is configured to bend in at least a first direction. The skin-coupling surface may be considered to be configured to bend if the curvature of the skin-coupling surface is able to change. In Fig. 5, the skin-coupling surface is configured to bend about an axis in the x-direction. In particular, the skin-coupling surface is configured to bend about a first axis x₁ and a second axis x₂, parallel to the first axis. In some examples, the skin-coupling surface may be configured to bend in two perpendicular directions (e.g. about one or more axes in an x-direction and about one or more axes in a y-direction).

The skin-coupling element 111 of Fig. 5 comprises first regions 116 of a first, low rigidity, positioned between second regions 117 of higher rigidity. The first regions are sufficiently flexible to allow the skin-coupling element to bend at the first regions. In other words, the first regions are elastically deformable elements that are configured to bend.

Four flexible first regions are provided in the skin-coupling element shown in Fig. 5, allowing the skin-coupling element to bend about the first and second axes x1, x2 on either side of the aperture 115; however, the skilled person will appreciate that a greater or smaller number of flexible first regions may be used.

In Fig. 5, the flexible first regions 116 are regions having a smaller thickness in a z-direction (i.e. a direction perpendicular to the skin-coupling surface) than the second regions. In particular, the thickness in the z-direction of the flexible first regions is such that a desired flexibility is achieved. The skilled person will appreciate that a thickness required to achieve a particular flexibility will depend on the material used for the flexible first regions, and will be readily capable of determining a suitable thickness for a particular resilient member.

Further examples of skin-coupling elements having a skin-coupling surface configured to bend in at least a first direction include skin-coupling elements in which flexible first regions are formed from a different material to the more rigid second regions, and skin-coupling elements comprising pivotable parts (i.e. parts configured to pivot with respect to one another).

The flexible first regions 116 (or other elements that enable bending of the skin-coupling surface) may be positioned in the skin-coupling element at locations corresponding to expected locations of high curvature of the skin during use of the device 100. For instance, during hemodynamic monitoring, an ultrasound transducer is typically placed at a desired angle with respect to the artery being imaged. The positions of the flexible first regions may correspond to expected locations of high skin curvature when the device is placed on a subject's skin in order to mount an ultrasound transducer at the desired angle.

Preferably, the skin-coupling element 111 is configured such that, when the device 100 is secured to the subject's skin, an angle between the skin-coupling surface and the surface of the skin to which the device is secured is zero, or close to zero in order to prevent or reduce a likelihood of peeling.

Fig. 6 illustrates a side view of the resilient member 110 in a deformed state. In Fig. 6, the skin-coupling element has bent at the first regions 116 to provide a skin-coupling surface 111a that conforms to the curvature of a subject's skin. Fig. 6 also illustrates the displacement between the skin-coupling element 111 and the inner rim 112 when the resilient member is deformed.

In some examples, the acoustic gel pad may comprise a skin-coupling pad surface that is curved to better conform to a skin curvature. Similarly, the housing for the acoustic gel pad may comprise a skin-coupling housing surface that is curved to better conform to a skin curvature. Since the skin curvature of an area of skin on which the device is mounted will vary greatly between subjects and between different body parts of the same subject, the device may be provided with a set of acoustic gel pads, each having a different curvature for the skin-coupling pad surface, and/or a set of housings, each having a different curvature for the skin-coupling housing surface, in order to allow an acoustic gel pad and/or housing having an appropriate curvature to be selected. The set(s) may include an acoustic gel pad and/or a housing with a flat skin-coupling surface.

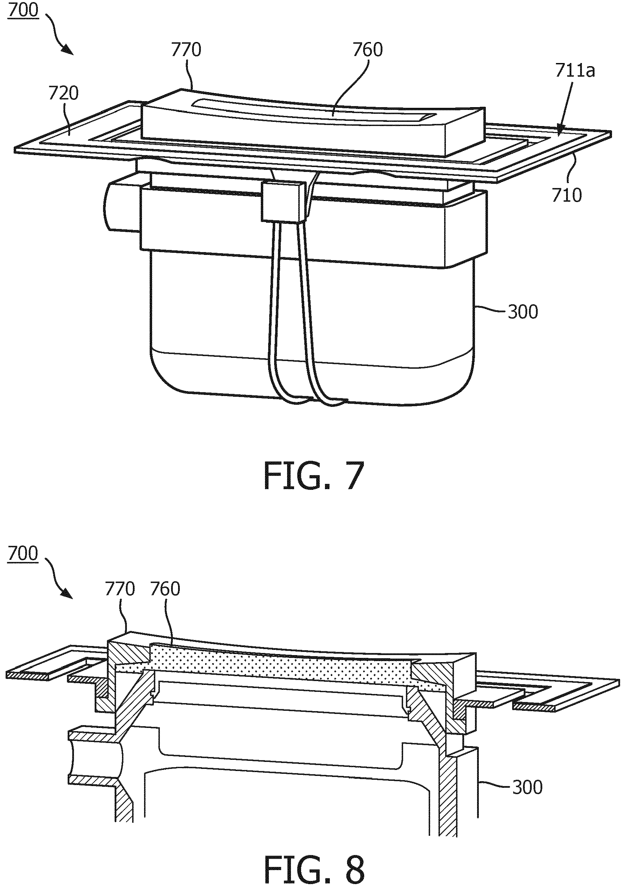

Fig. 7 illustrates a perspective side view of a device 700 for mounting a sensor on a subject's skin, according to an embodiment of the invention. The ultrasound transducer is mounted in the device.

The device 700 is similar to the device 100 illustrated in Fig. 1, but the acoustic gel pad 760 comprises a curved skin-coupling pad surface, and the housing 770 for the acoustic gel pad comprises a curved skin-coupling housing surface. The second adhesive layer is not included in device 700.

Fig. 7 also illustrates an offset, in a direction perpendicular to the skin-coupling pad surface, between the skin-coupling surface 711a of the resilient member 710 and the skin-coupling pad surface when the resilient member is in an undeformed state. In particular, the skin-coupling surface of the resilient member is offset from the skin-coupling pad surface by a first distance of about 5 mm .

This offset means that, when the device 700 is placed on a subject's skin with the resilient member 710 in an undeformed state, the first adhesive layer 720 (and second adhesive layer, if present) does not contact the subject's skin. This allows the device to be moved across a subject's skin, to achieve a desired position of the device with respect to the subject's skin, without the device adhering to the skin.

In some examples, a backing layer may be provided on the adhesive surface of the at least one adhesive layer. The offset allows the backing layer to be removed from the at least one adhesive layer, once a desired position has been achieved, without removing the device 700 from the subject's skin.

Once the device 700 has a desired position on a subject's skin, the skin-coupling element of the resilient member 710 may be pressed towards the skin, deforming the resilient member to create a preload force between the device and the subject's skin. By pressing the skin-coupling element to the skin, the first adhesive layer 720 is brought into contact with the skin, and adheres to the skin, thus securing the device to the skin.

Therefore, in the deformed state of the resilient member 710, the offset, in a direction perpendicular to the skin-coupling pad surface, between the skin-coupling surface 711a of the resilient member and the skin-coupling pad surface is the thickness of the first adhesive layer 720 in the direction perpendicular to the skin-coupling pad surface. In examples in which the device does not include the first adhesive layer, the offset in the deformed state may be zero (i.e. the skin-coupling surface of the resilient member may directly contact the skin).

Fig. 8 illustrates a sectional view of the device 700. As can be seen from Fig. 8, while the skin-coupling pad surface is curved, a surface of the acoustic gel pad 760 that contacts the ultrasound transducer 300 is flat. As the ultrasound-emitting surface of the ultrasound transducer is flat, a flat sensor-coupling pad surface provides better acoustic contact. In other examples, the acoustic gel pad may comprise a curved sensor-coupling pad surface to complement a different sensor geometry (e.g. an ultrasound transducer with a curved ultrasound-emitting surface).

In some examples, the acoustic gel pad and the housing for the acoustic gel pad may be configured to interlock to secure the acoustic gel pad to the housing. Acoustic gel pads cannot be attached to a housing with glue , so the use of interlocking geometry provides a simple way to secure the acoustic gel pad to the housing, reducing a likelihood that the acoustic gel pad will be pushed out of the housing during mounting of the device.

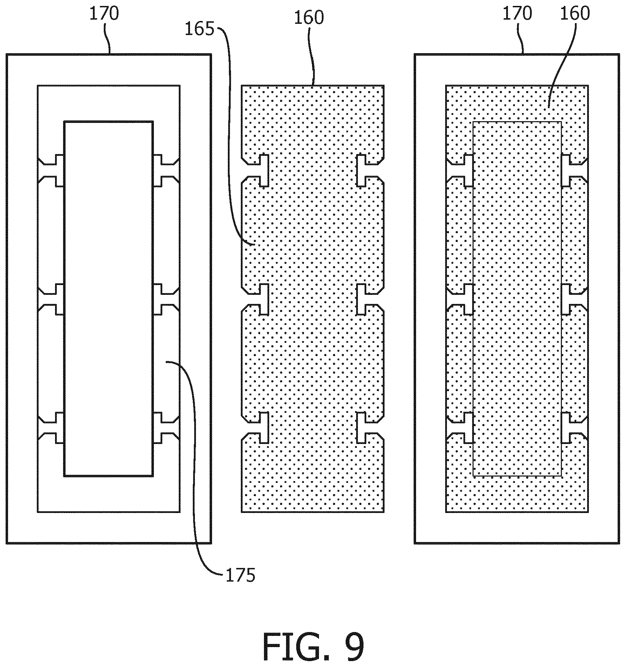

Fig. 9 illustrates a top view of the acoustic gel pad 160 and the housing 170 disposed side by side, and a top view of the acoustic gel pad secured within the housing. The acoustic gel pad comprises a series of interlocking elements 165. The inside of the housing 170 is dimensioned to provide a friction fit for the acoustic gel pad, and comprises a series of grooves 175. The grooves are positioned at the locations of the interlocking elements when the acoustic gel pad is contained within the housing, and have complementary geometry to the interlocking elements, such that each interlocking element engages with a respective groove.

The acoustic gel pad 160 may be made by pouring a suitable gel composition, in its liquid state, into a mold having the same shape as the housing 170. The gel composition will solidify in the mold at room temperature to form an acoustic gel pad having the desired shape.

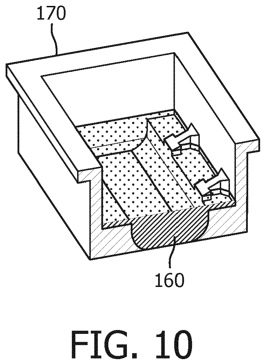

Fig. 10 illustrates a sectional view of the acoustic gel pad 160 secured within the housing 170.

Although the example devices shown in the Figures include an acoustic gel pad and housing, the skilled person will appreciate that, in some examples, the device may not include the acoustic gel pad and housing. For instance, when the device is used to mount a sensor other than an ultrasound transducer on a subject, the sensor may directly contact the subject's skin.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device (100, 700) for mounting a sensor (300) on a subject's skin, the device comprising:
a resilient member (110, 710) configured to deform to create a preload force between the device and the subject's skin, wherein the resilient member comprises a skin-coupling element (111) having a skin-coupling surface (111a, 711a) configured to bend in at least a first direction;
at least one adhesive layer (120, 130, 720) for securing the device to the subject's skin; and
a sensor-securing arrangement (140, 150) configured to secure a sensor to the subject's skin when the device is secured to the subject's skin.

2. The device (100, 700) of claim 1, wherein the skin-coupling element (111) comprises at least one first region (116) of a first rigidity and two or more second regions (117) of a second rigidity higher than the first rigidity, wherein each first region is positioned between at least two of the second regions.

3. The device (100, 700) of claim 2, wherein:
each first region (116) is formed by a region of the skin-coupling element (111) having a first thickness in a direction (z) perpendicular to the skin-coupling surface of the skin-coupling element; and
each second region (117) is formed by a region of the skin-coupling element having a second thickness in the direction perpendicular to the skin-coupling surface of the skin-coupling element, wherein the second thickness is greater than the first thickness.

4. The device (100, 700) of any of claims 1 to 3, wherein the skin-coupling element (111) is configured such that, when the device is secured to the subject's skin, an angle between the skin-coupling surface (111a, 711a) and the surface of the subject's skin to which the device is secured is zero.

5. The device (100, 700) of any of claims 1 to 4, wherein the skin-coupling surface (111a, 711a) is further configured to bend in a second direction, perpendicular to the first direction.

6. The device (100, 700) of any of claims 1 to 5, wherein the resilient member (110, 710) comprises a plurality of interconnected ribs extending around an aperture (115), wherein the resilient member is configured to deform by moving between an undeformed state, in which the interconnected ribs are closer together, and a deformed state, in which the interconnected ribs are further apart.

7. The device (100, 700) of any of claims 1 to 5, wherein the resilient member comprises a pleated structure comprising a plurality of pleats, wherein the pleated structure is configured to deform by moving between an undeformed state, in which the plurality of pleats are contracted, and a deformed state, in which the plurality of pleats are expanded.

8. The device (100, 700) of any of claims 1 to 7, wherein the at least one adhesive layer comprises a first adhesive layer (120) on the skin-coupling surface (111a, 711a) of the skin-coupling element (111).

9. The device (100, 700) of any of claims 1 to 8, wherein the at least one adhesive layer comprises a second adhesive layer (130) provided around an outer periphery of the skin-coupling surface (111a, 711a) of the skin-coupling element (111).

10. The device (100, 700) of any of claims 1 to 9, wherein the device further comprises:
an acoustic gel pad (160, 760) comprising a skin-coupling pad surface; and
a housing (170, 770) for holding the acoustic gel pad.

11. The device (700) of claim 10, wherein the skin-coupling pad surface is curved.

12. The device (700) of claim 10 or 11, wherein the housing (770) comprises a skin-coupling housing surface, wherein the skin-coupling housing surface is curved.

13. The device (100, 700) of any of claims 10 to 12, wherein:
the at least one adhesive layer (120, 130) is attached to the resilient member (110, 710);
in an undeformed state of the resilient member, the skin-coupling surface (111a, 711a) of the resilient member is offset from the skin-coupling pad surface in a direction perpendicular to the skin-coupling pad surface by a first distance; and
in a deformed state of the resilient member, an offset of the skin-coupling surface of the resilient member and the skin-coupling pad surface in a direction perpendicular to the skin-coupling pad surface is less than the first distance.

14. The device (100, 700) of claim 13, wherein, in a deformed state of the resilient member (110, 710), the offset of the skin-coupling surface (111a, 711a) of the resilient member and the skin-coupling pad surface in a direction perpendicular to the skin-coupling pad surface is zero.

15. The device (100, 700) of any of claims 10 to 14, wherein the acoustic gel pad (160, 760) and the housing (170, 770) are configured to interlock to secure the acoustic gel pad to the housing.
